# EUROPÄISCHE PATENTSCHRIFT

(11) **EP 3 355 992 B1**
(45) Veröffentlichungstag und Bekanntmachung des Hinweises auf die Patenterteilung: **16.12.2020**
(21) Anmeldenummer: 16793737.4
(22) Anmeldetag: 30.09.2016
(51) Int. Cl.: A61K 31/045, A61K 36/185, A61K 36/899, A61Q 5/00, A61Q 7/00, A61K 8/00, A61P 17/14, A61K 8/9789

(54) **HAARWUCHSMITTEL SOWIE VERFAHREN ZU SEINER HERSTELLUNG UND VERWENDUNG DES MITTELS ZUR PROPHYLAKTISCHEN BEHANDLUNG VON HAARAUSFALL**
HAIR GROWTH AGENT, METHOD FOR THE PRODUCTION THEREOF, AND USE OF THE AGENT FOR THE PROPHYLACTIC TREATMENT OF HAIR LOSS
AGENT DE CROISSANCE CAPILLAIRE, PROCÉDÉ POUR LE PRÉPARER ET UTILISATION DE L'AGENT POUR LE TRAITEMENT PROPHYLACTIQUE DE L'ALOPÉCIE

(30) Priorität: 02.10.2015 DE 102015116746
(43) Veröffentlichungstag der Anmeldung: 08.08.2018
(73) Patentinhaber: Seyda, Stefanie, 58540 Meinerzhagen (DE)
(72) Erfinder: Seyda, Stefanie, 58540 Meinerzhagen (DE)
(74) Vertreter: Christophersen Patentanwälte
(86) Internationale Anmeldenummer: PCT/DE2016/100460
(87) Internationale Veröffentlichungsnummer: WO 2017/054806

(56) Entgegenhaltungen:
- EP-A1- 2 772 245
- EP-A2- 1 752 137
- DE-A1-102006 050 398
- FR-A1- 2 953 722
- US-A1- 2010 247 428
- US-A1- 2013 064 908
- DATABASE GNPD [Online] MINTEL; Mai 2014 (2014-05), "Black Hair essence", XP002766346, Database accession no. 2361888
- Ji Young Oh, Min Ah Park, Young Chul Kim: "Peppermint Oil Promotes Hair Growth without Toxic Signs", Toxicological research, Bd. 30, Nr. 4 26. Dezember 2014 (2014-12-26), Seiten 297-304, XP002766347, DOI: 10.5487/TR.2014.30.4.297 Gefunden im Internet: URL:http://ocean.kisti.re.kr/downfile/volu me/ksot/DSHHBQ/2014/v30n4/DSHHBQ_2014_v30n 4_297.pdf [gefunden am 2017-01-13]

## Beschreibung

Die vorliegende Erfindung betrifft ein Haarwuchsmittel sowie ein Verfahren zu seiner Herstellung und die Verwendung des Mittels zur prophylaktischen Behandlung von Haarausfall.

Haarausfall ist ein weitverbreitetes Phänomen, das sowohl bei Personen männlichen und weiblichen Geschlechts auftritt und zur Alopezie führen kann. Gründe für den Haarausfall sind u. a. genetische Veranlagung, der Einfluss insbesondere androgener Hormone, Infektionen im Haarwurzelbereich oder der Kopfhaut, durch Medikamenteneinwirkung, aber auch mechanische Einwirkung. Ferner können auch Infektionskrankheiten, Stress und Intoxikationen zu Haarausfall führen.

Aber auch Witterungseinflüsse und/oder eine häufig ungesunde Lebensführung schlagen sich zunehmend in der Widerstandskraft der Haarwurzeln nieder, u. a. trocknet die Kopfhaut aus, spannt und verliert an Elastizität und in der Konsequenz auch an Haaren.

Kahle Stellen auf dem Kopf, Geheimratsecken oder Glatzen gelten als unattraktiv. Im Handel wird eine Vielzahl von Haarwuchsmitteln angeboten, die das gesunde, dichte Haar erhalten sollen. Dieser Aufgabe werden jedoch kaum Mittel gerecht und wenn Erfolge zu verzeichnen sind, so nur durch teure Produkte, die Aktivstoffe enthalten, die nicht frei von Nebenwirkungen sind und die auch nur wirksam bei erblichem, hormonell bedingtem Haarausfall sind (z. B. Minoxidil).

Bei diffusem Haarausfall werden Produkte empfohlen, die Zystin und B-Vitamine enthalten. Diese Bestandteile sind jedoch lediglich geeignet, die Gesundheit von nachwachsender Haut, Haaren und Fingernägeln zu fördern, jedoch nicht, Glatzen oder kahle Stellen auf dem Kopf wieder mit Haar zu füllen.

US2010/0247428 betrifft ein Haarwuchsmittel enthaltend als aktive Komponente, selten Erdelemente sowie Praseodym, Promethium, Europium, Terbium, Dysprosium, Thulium, Yttrium, Lanthan, Samarium, Gadolinium, Ruthenium und Gemischen der voranstehenden, Salze oder Oxide dieser Elemente.

Eine Vielzahl von Hilfsstoffen, die in dem Mittel enthalten sein können, sowie Mungbohne und Menthol.

Es besteht nach wie vor ein Bedart an Mitteln, die den Haarwuchs tordern und so Glatzen und kahle Stellen auf dem Kopf wieder mit Haar gefüllt werden, und auch zur prophylaktischen Behandlung von Haarausfall geeignet sind.

Gegenstand der vorliegenden Erfindung ist demgemäß ein Haarwuchsmittel auf der Basis einer alkoholischen, wässrigen und/oder wässrig-alkoholischen Lösung enthaltend
A) 0,001 bis 0,99 Gew.-Anteile, bezogen auf Gewichtsteile des fertigen Mittels, Menthol
B) 0,1 bis 3 Gew.-Anteile, bezogen auf 100 Gewichtsteile des fertigen Mittels, Bambus-Extrakt oder Presssaft aus Bambus
C) 5 bis 10 Gew.-Anteile, bezogen auf 100 Gewichtsteile des fertigen Mittels, Hülsenfrüchte-Extrakt oder Presssaft von Hülsenfrüchten
D) 2 bis 10 Gew.-Anteile, bezogen auf 100 Gewichtsteile des fertigen Mittels, Kräuterextrakte oder Presssaft aus Kräutern.

Es wurde überraschenderweise gefunden, dass bei der Applikation eines Mittels enthaltend die oben genannten Wirkstoffe A bis D das Haarwachstum bei Menschen, die unter Haarausfall leiden oder kahle Stellen auf dem Kopf haben, deutlich verbessert werden kann und kahle Stellen deutlich verringert werden können.

Das erfindungsgemäße Haarwuchsmittel liegt üblicherweise in Form einer Kombination der unter A, B, C und D genannten Wirkstoffe in Form einer wässrigen, alkoholischen oder wässrig-alkoholischen Lösung vor. Darüber hinaus können noch weitere Alkohole, die beispielsweise als Komponenten oder Extraktionsmittel der Komponenten C und/oder D dem erfindungsgemäßen Mittel zugeführt werden, enthalten sein.

Als Komponente A enthält das erfindungsgemäße Mittel Menthol. Das Menthol kann als Enantiomeren-Gemisch oder in Form eines reinen Stereoisomeren eingesetzt werden, wie (-)-Menthol, (+)-Menthol, (+)-Isomenthol, (-)-Isomenthol, (+)-Neomenthol, (-)-Neomenthol, (+)-Neoisomenthol, (-)-Neoisomenthol sowie in beliebigen Gemischen der voranstehenden. Vorzugsweise wird das auch in vielen ätherischen Ölen vorkommende (-)Menthol (Levomenthol) eingesetzt. Vorzugsweise ist Menthol in dem erfindungsgemäßen Mittel in einer Menge von 0,001 bis 0,99 Gew.-Anteile, insbesondere von 0,005 bis 0,99 Gew.-Anteile, bezogen auf 100 Gewichtsteile des fertigen Mittels, enthalten.

Ein weiterer Bestandteil ist ein Presssaft bzw. Extrakt aus Bambus (Komponente B). Bevorzugt werden wässrige Extrakte oder wässrig-alkoholische Extrakte oder wässrigorganische oder alkoholische Extrakte von Bambus eingesetzt. Vorzugsweise wird im Handel erhältlicher und für die topische Anwendung geeigneter Bambus-Extrakt oder Presssaft aus Bambus in dem erfindungsgemäßen Mittel in einer Menge von 0,1 bis 3 Gew.-Anteile, bezogen auf 100 Gewichtsteile des fertigen Mittels, verwendet.

Die Komponente C ist Presssaft oder Extrakte von Hülsenfrüchten. Hülsenfrüchte zeichnen sich durch ihren hohen Proteingehalt aus. Geeignete Hülsenfrüchte, deren Presssaft oder Extrakte im Rahmen der vorliegenden Erfindung eingesetzt werden können, sind Bohnen, Erbsen, Erdnüsse, Ginster, Kichererbsen, Klee, Linsen, Lupinen, Sojabohnen, und/oder Wicke. Auch Gemische von Presssäften und Extrakten von verschiedenen Hülsenfrüchten können eingesetzt werden. Die Komponente C ist in dem erfindungsgemäßen Mittel vorzugsweise in einer Menge von 5 bis 10 Gew.-Anteilen, bezogen auf 100 Gewichtsteile des fertigen Mittels, enthalten.

Als Komponente D enthält das erfindungsgemäße Mittel einen Extrakt oder Presssaft aus Kräutern. Als geeignete Kräuter können Angelikawurzel, Andornkraut, Basilikum, Lavendel, Oregano, Quendel, Rosmarin und/oder Thymian genannt werden. Die Komponente D wird vorzugsweise in Form eines alkoholischen oder wässrig-alkoholischen Kräuterextrakten eingesetzt. Vorzugsweise wird die Komponente D in Form eines handelsüblichen Extrakts oder Presssaftes in einer Menge von 2 bis 10 Gew.-Anteilen, bezogen auf 100 Gewichtsteile des fertigen Mittels, verwendet.

Die Extrakte können mit Wasser sowie polaren oder unpolaren organischen Lösungsmitteln sowie Mischungen davon in dem Fachmann bekannter Weise hergestellt werden. Extrakte, die durch Extraktion mit einem Alkohol oder Wasser/Alkohol-Mischungen erhalten werden können, sowie Presssaft, sind bevorzugt. Als Alkohole können ein- oder mehrwertige Alkylalkohole verwendet werden, beispielsweise Methanol Ethanol, Propanol, i-Propylalkohol, Glykol, Propylenglykol und/oder Glycerin,

Es können sowohl die Extrakte im ursprünglichen Extraktionsmittel als auch Extrakte/Presssaft in Wasser oder anderen organischen Lösungsmitteln und/oder deren Gemisch, insbesondere Ethanol sowie Ethanol/Wasser-Mischungen, eingesetzt werden.

Es ist nicht unbedingt erforderlich, frisch hergestellte Presssäfte oder Extrakte einzusetzen, auch im Handel erhältliche Presssäfte oder Extrakte sind im Rahmen der vorliegenden Erfindung geeignet.

Die Komponenten B, C und D sind insgesamt vorzugsweise in einer Menge von 3 bis 20 Gew.-Anteilen, bezogen auf 100 Gewichtsanteile, insbesondere in einer Menge von 3 bis 20 Gew.-Anteilen, bezogen auf 100 Gewichtsanteile, des fertigen Mittels, enthalten. Die eingesetzten Pflanzenextrakte sind im Handel erhältliche Produkte, die üblicherweise die pflanzlichen Inhaltsstoffe in einer Konzentration zwischen 0,005 und 0,05 Gew.-%, insbesondere zwischen 0,01 und 0,03 Gew.-% in einer wässrigen, alkoholischen oder wässrig-alkoholischen Lösung enthalten.

Die Anwendung des erfindungsgemäßen Mittels erfolgt üblicherweise topisch, wobei das Mittel einfach ins Haar, auf die Kopfhaut bzw. auf Hautstellen, auf denen der Haarwuchs seit Längerem eingestellt ist bzw. der Haarausfall besonders deutlich ist und dort wieder reaktiviert werden soll, durch Sprühen, Auftröpfeln, Einmassieren, Auftragen und/oder Kneten appliziert wird. Nach Applikation des Mittels ist es zumeist nicht notwendig, das Haar bzw. die Kopfhaut noch einmal auszuspülen oder mit weiteren zusätzlichen Mitteln zu behandeln, um eine positive Wirkung zu erreichen. Das erfindungsgemäße Mittel kann auf dem Haar verbleiben.

Das erfindungsgemäße Mittel kann in jeder, für die Behandlung von Haaren und/oder Kopfhaut geeigneten Zubereitung vorliegen bzw. in einer solche Zubereitung eingearbeitet werden, insbesondere als Shampoo, Haarspülung, Haarkur, Gel, Schaum, Haaröl, Spray und/oder Haarcreme. Haarcreme eignet sich insbesondere für prophylaktische Behandlung. Mittel, die zur prophylaktischen Behandlung verwendet werden, können die oben genannten Inhaltsstoffe in einer geringeren Konzentration enthalten als Mittel, die unmittelbar der Behandlung von Haarausfall oder von kahlen Stellen am Kopf eingesetzt werden.

Das erfindungsgemäße Mittel liegt besonders bevorzugt als Haarserum/Haartonikum vor, das nach der Anwendung auf dem Haar verbleiben kann. Eine solche Formulierung wird vorzugsweise bei Raumtemperatur angewendet. Das Tonikum/Serum enthält ein Gemisch aus Wasser und Ethanol, wobei der Gehalt an Ethanol vorzugsweise im Bereich von etwa 8 % bis 25 % liegt. Der pH-Wert des erfindungsgemäßen Mittels liegt vorzugsweise im sauren Bereich, insbesondere zwischen 4,5 und 6,5. Zur Herstellung des pH-Wertes kann jede für kosmetische Zwecke verwendbare Säure oder Base verwendet werden. Üblicherweise werden Säuren verwendet, die auch im Rahmen der üblichen Nahrungsaufnahme aufgenommen werden und positive Auswirkungen auf den menschlichen Organismus haben. Die Säuren können auch in Kombination mit weiteren Komponenten als Säure-Puffer wirken. Beispiele für geeignete Säuren sind Essigsäure, Milchsäure, Weinsäure, Zitronensäure, Äpfelsäure, Ascorbinsäure und Gluconsäure. Im Rahmen der vorliegenden Erfindung ist die Verwendung von Zitronensäure und Milchsäure besonders bevorzugt.

Als weitere Wirk-, Hilfs- und Zusatzstoffe kann das erfindungsgemäße Mittel Tenside, beispielsweise nichtionische, anionische, zwitterionische und ampholytische Tenside, nichtionische Polymere, Konservierungsmittel, Verdickungsmittel, Strukturantien, haarkonditionierende Verbindungen, Vitamine und Provitamine, Duftstoffe, wie Parfümöle, pH-Stabilisatoren, UV-Absorber sowie Lösungsmittel und Lösungsvermittler sowie faserstrukturverbessernde Wirkstoffe, Anti-Schuppen-Wirkstoffe, Lichtschutzmittel, Konsistenzgeber, Fette und Wachse, Komplexbildner, Quell- und Penetrationsstoffe usw. enthalten.

Als weitere pflanzliche Zusatzstoffe, die in Ergänzung zu den Komponenten A bis D enthalten sein können, können grüner Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Konjakwurzel, Sandelholz, Wacholder, Kokosnuss, Mango, Aprikose, Limone, Weizen, Kiwi, Melone, Orange, Grapefruit, Salbei, Birke, Malve, Wiesenschaumkraut, Schafgarbe, Melisse, Hauhechel, Huflattich, Eibisch, Meristem, Ginseng und Ingwerwurzel in Form von Extrakten, Presssäften oder auch in Form von Pulvern oder in sonstiger Form eingesetzt werden.

Ein weiterer Gegenstand der vorliegenden Erfindung ist ein Verfahren zur Herstellung des erfindungsgemäßen Mittels, welches die Verfahrensschritte umfasst
a) Herstellen einer Lösung aus Menthol in Wasser, Alkohol oder einem Gemisch aus Wasser und Alkohol,
b) Einstellen des pH-Werts auf einen Bereich zwischen 4,5 und 6,5,
c) Hinzufügen der Inhaltsstoffe der Komponenten C und D und ggf. weiterer Komponenten unter Rühren.

Das erfindungsgemäße Verfahren zur Herstellung des Haarwuchsmittels wird vorzugsweise bei Raumtemperatur, d. h. in der Regel bei 20 °C, durchgeführt.

In einer weiteren möglichen Ausführungsform enthält das erfindungsgemäße Haarwuchsmittel auf der Basis einer alkoholischen, wässrigen und/oder wässrig-alkoholischen Lösung die Komponenten B, C und D,
B) Bambus-Extrakt oder Presssaft aus Bambus
C) Hülsenfrüchte-Extrakt oder Presssaft von Hülsenfrüchten
D) Kräuterextrakte oder Presssaft aus Kräutern.

In dieser Ausführungsform wird auf den Zusatz von Menthol verzichtet. Die Komponenten B, C und D sowie die weiteren Inhaltsstoffe können in den gleichen Mengen, wie sie oben zu einer Zusammensetzung aus den Komponenten A, B, C und D angegeben sind, in dem Mittel enthalten sein.

### Beispiele

### Zubereitung 1 (Mengenangaben jeweils an Gewichtsteile)

| | | |
|---|---|---|
| 61,740 | Wasser | Aqua |
| 18,000 | Alkohol | Alcohol denat. |
| 7,000 | Pflanzenextrakt Sojabohne, Kichererbse, Linse | Aqua, Alkohol denat. Glycine Max (Soybean) Seed Extract Cicer Arietinum Seed Extract, Lens Esculenta (Lentil) Seed Extract |
| 5,000 | Pflanzenextrakt Angelika, Andorn, Thymian | Aqua, Propylene Glycol, Angelica Archangelica Extract, Marrubium Vulgare Extract, Thymus Vulare (Thyme) Extrakt) |
| 2,000 | Nutrilan® Keratin W PP | Hydrolyzed Keratin |
| 1,000 | Cremophor RH 410 | PEG-40 Hydrogenated Castor Oil |
| 1,000 | Kamillenextrakt | Chamomilla Recutita Flower Extract |
| 1,000 | Brennessel-Extrakt | Aqua, Alcohol, Urtica Dioica ( Nettle) Leaf Extract |
| 1,000 | Bambus Extrakt GW | Aqua, Glycerin, Bambusa Vulgaris Leaf Extract |
| 0,800 | Dermosoft ® decalact deo | Sodium Caproyl/Lauroyl Lactylate; Triethylcitrate; Salvia Officinalis (Sage) Oil |
| 0,500 | Panthenol | Panthenol |
| 0,200 | Naturex C 100 | Polyquaternium 37, Propylene Glycol Dicaprylate/Dicaprate, PEG-1 Trideceth 6, Hydroxyethylcellulose |
| 0,200 | PÖ Cucumber-Melon | Parfum |
| 0,200 | Plantacare 818 | Coco-Glucoside |
| 0,100 | Aloe Vera freezed dried powder 200:1 | Aloe Barbadensis Leaf Juice Pwder |
| 0,100 | Konjak Mannan Gel Powder | Amorphophallus Konjac Root Powder |
| 0,100 | Uvinul MS 40 | Benzophenone-4 |
| 0,020 | Menthol | Menthol |
| 0,010 | Milchsäure | Lactic Acid |
| 0,010 | Lactose | Milchzucker |
| 0,010 | Äpfelsäure | Malic Acid |
| 0.010 | Zitronensäure | Citric Acid |

Zur Herstellung der Zubereitung 1 wird zunächst eine klare Vorlösung aus Menthol und Alkohol hergestellt. Anschließend wird das auf 20 °C vortemperierte Wasser zugefügt. Der pH-Wert der erhaltenen Lösung wird mit Zitronensäure auf 5,0 eingestellt. Die weiteren Inhaltsstoffe werden nacheinander separat und unter ständigem Rühren zu der Lösung zugegeben. Der pH-Wert der fertigen Lösung ist 5,0, die Viskosität bei 20 °C 1,00 mPa·s.

### 1. Wirkung auf Haarausfall

Ein Serum gemäß Zubereitung 1 wurde insgesamt 30 Probanden (20 Männer und 10 Frauen) mit einem durchschnittlichen Haarausfall von 100 Haaren pro Tag über einen Zeitraum von drei Monaten appliziert. Die Probanden waren zwischen 25 und 65 Jahre alt.

Es wurden zweimal täglich je 0,5 ml der Zusammensetzung aufgetragen.

Testparameter war die Reduzierung des täglich ausgefallenen Haares, was durch Zählen der ausgefallenen Haare quantifiziert wurde.

Die Probanden wurden willkürlich in zwei Gruppen aufgeteilt. Einer Gruppe von 15 Probanden (10 Männer und 5 Frauen) wurde das Serum gemäß obigem Beispiel verabreicht, die zweite Gruppe von 15 Probanden (10 Männer und 5 Frauen) verwendete ein Placebo, d. h. ein Produkt ohne Wirkung.

Zu Beginn der Studie, an den Tagen 0 bis 3, wurde die Zahl der ausgefallenen Haare gezählt, diese Haare wurden in Briefumschlägen abgegeben. Das Zählen der Haare erfolgte während der drei Tage durch morgendliches und abendliches Kämmen des Haares und Sammeln der Haare aus dem Kamm.

Über 30 Tage wurde zweimal täglich, morgens und abends, das Serum appliziert. An den Tagen 27 bis 30 sammelten die Probanden die beim Kämmen verlorenen Haare aus der Bürste.

Am Tag 31 wurden die Haare gezählt. Das Sammeln der Haare erfolgte noch zweimal während der Tage 57 bis 60 und 87 bis 90. An den Tagen 61 und 91 wurde das gesammelte Haar gezählt.

### Ergebnisse

Die Gruppe A, die die Kopfhaut mit dem Serum gemäß obigem Beispiel behandelt hatte, zeigte eine deutliche Reduzierung des Haarausfalls, bereits nach einem Monat der Behandlung mit dem Serum sank der Haarausfall um 28 %, nach zwei Monaten um 33 % und nach drei Monaten um 38 %.

Die Gruppe B, die ein Placebo anwendete, zeigte keine Verringerung des Haarausfalls.

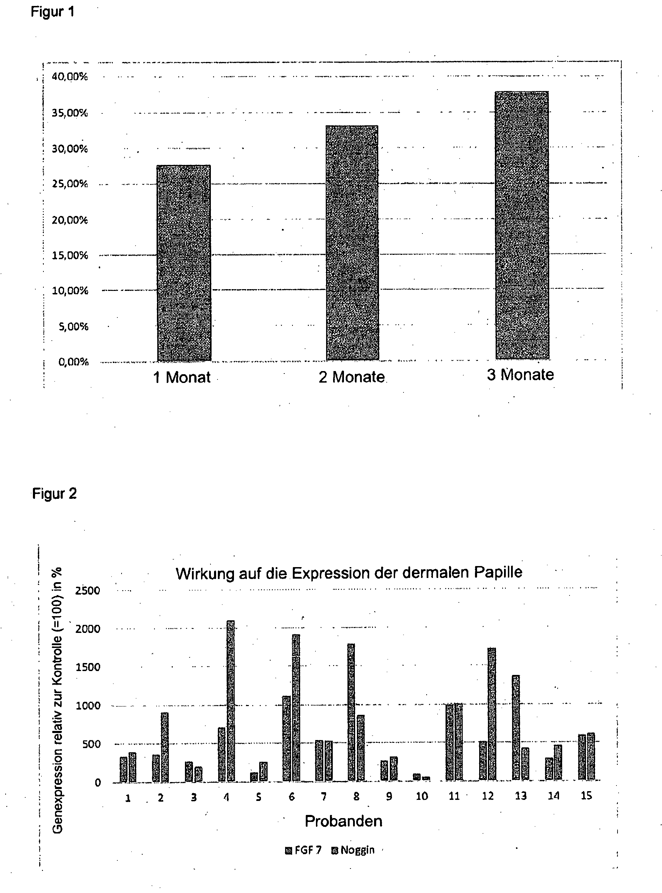

Die Ergebnisse des Beispiels 1 sind in Figur 1 dargestellt.

### Beispiel 2 Wirkung auf das Haarwachstum

Ein Serum gemäß Zubereitung 1 wurde insgesamt 30 Probanden (20 Männer und 10 Frauen) mit einem durchschnittlichen Haarausfall von 100 Haaren pro Tag über einen Zeitraum von drei Monaten appliziert. Die Probanden waren zwischen 25 und 65 Jahre alt.

Das Serum wurde zweimal täglich, morgens und abends, aufgetragen.

Die Probanden wurden willkürlich in zwei Gruppen aufgeteilt. Einer Gruppe von 15 Probanden (10 Männer und 5 Frauen) wurde das Serum gemäß obigem Beispiel verabreicht, eine zweite Gruppe von 15 Probanden verwendete ein Placebo, d. h. ein Produkt ohne Wirkung.

### Durchführen der Untersuchung:

Die Probanden wurden angewiesen, keine Haarmittel, die Östrogene enthielten, Therapeutika mit Einfluss auf das Haarwachstum und Haarshampoo, das Koffein enthielt, bis zum Abschluss der Studie zu verwenden.

### Tag 1

### Bestimmung aller Parameter

Vor der Behandlung wurden 20 Haare mit Haarwurzel aus der definierten Fläche der Kopfhaut entfernt, gemeinsam in ein RNA-Röhrchen gegeben und sofort auf -80 °C tiefgefroren (T₀ = Kontrollprobe).

Das Serum gemäß obigem Beispiel wurde jeweils zweimal am Tag, jeweils morgens und abends, auf die definierte Zone aufgebracht. Nach 7 und nach 21 Tagen wurden jeweils 20 Haare mit Haut von der definierten Kopfhautfläche gezupft, in ein RNAse-freies Röhrchen gegeben und sofort auf-80 °C gefroren (T₁).

Die gesammelten Proben T₀ und T₁ wurden auf ihre Genexpressionsgeschwindigkeit unter Verwendung von RT-PCR analysiert. Die selektierten Gene sind wichtig für das Haarwachstum, die Haarpigmentierung und den Glanz des Haares.

### Ergebnisse

Nach 21 Tagen Behandlung mit dem Serum wurden die Gene untersucht, die wichtig für die Induzierung der Wachstumsphase der Haarfollikel und der Entwicklung des Haars sind. Das Ergebnis zeigt, dass die Wirkung auf die Genexpression die Wirkung auf das Haarwachstum nicht vom Geschlecht oder Alter abhängt.

Gruppe B zeigte keine Wirkung auf die Genexpression.

Die Ergebnisse sind in Figur 2 grafisch dargestellt.

### 3. Wirkung auf die Zellproliferation der dermalen Papille

4%iges Serum gemäß Zubereitung 1, das Extrakt gemäß Zubereitung 1 wurde lyophilisiert und in DMSO resuspendiert.

Untersucht wurden humane Zellen der dermalen Papille (hDPC). Testparameter waren VEGF (vaskulärer endotelialer Wachstumsfaktor) der als positive Kontrolle der Zellproliferation verwendet wurde, sowie MTT-(3-(4,5-Dimethyltiazol-2-yl)-2,5-Diphenyltetrazoliumbromid)-Assay. Die Zellen wurden über 18 Stunden im Serum ausgehungert, um die Zellproliferation zu stoppen und mit der Testsubstanz gemäß Zubereitung 1 über 72 Stunden inkubiert. Danach wurde das Gemisch zu einer Nährlösung von MTT:DMEM (1 : 2) gegeben und die Zellen wurden vier Stunden bei 37 °C bei Dunkelheit inkubiert. Die Reaktion wurde gestoppt, der Überstand wurde entfernt, DMSO zugefügt und für weitere zehn Minuten inkubiert. Anschließend wurde die Absorption bei 550 nm gemessen. Die Absorption von unbehandelten Zellen wurde als 100%ige Lebensfähigkeit (Kontrolle) gesetzt.

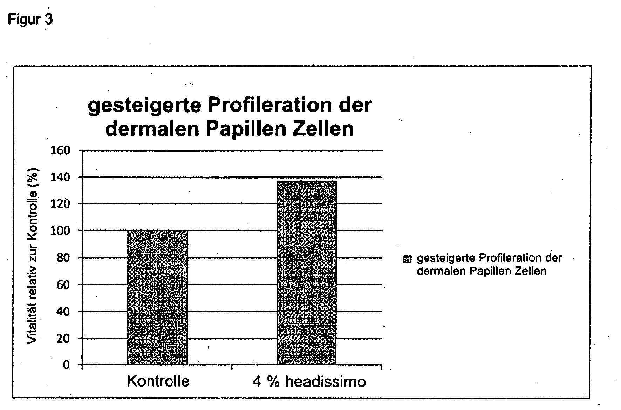

Es wurde festgestellt, dass das Serum gemäß Zubereitung 1 signifikant die Proliferation von Zellen der dermalen Papille stimuliert. Die grafische Darstellung des Ergebnisses aus Beispiel 3 ist in Figur 3 wiedergegeben.

### Beispiel 4 Wirkung auf den Haarausfall dargestellt mit einer Fototrichogramm-Analyse.

Die Wirkung auf den Haarausfall der Zubereitung 1 wurde unter Verwendung der Fototrichogramm-Technik aufgezeichnet.

Die Anagenphase ist die aktive Wachstumsphase der Haarfollikel. Die Haarwurzelzelle teilt sich schnell. Die telogene Phase ist die Ruhephase der Haarfollikel. Am Ende der telogenen Phase wird das Haar abgeworfen und das Haarfollikel tritt erneut in die anagene Phase ein. Die Haardichte, der Anteil des Haars in der anagenen und der telogenen Phase und der Wachstumskoeffizient (A/T) wurde bestimmt.

Ein definierter Bereich der Kopfhaut am Hinterkopf von einer Fläche von etwa 2 cm² wurde über 20 Wochen zweimal täglich, morgens und abends, mit der Zubereitung 1 behandelt. Die Versuchsgruppe bestand aus 17 Personen im Alter zwischen 25 und 65, davon waren zehn Personen männlich und sieben Personen weiblich.

Die Versuchspersonen befanden sich jeweils in einer Gruppe von ≥ 20 % in der telogenen Phase und wiesen eine Haardichtung von ≥ 150 Haaren/cm² auf.

### Durchführung der Untersuchung

Am ersten Tag kamen die Probanden mit frisch gewaschenem Haar. Nach dem Shampoonieren wurden keine zusätzlichen Produkte aufgebracht. Es wurden alle Parameter bestimmt und die zu untersuchende Fläche am Hinterkopf identifiziert. Das Haar wurde an der definierten Fläche rasiert und unter Verwendung eines Fotomikroskops Nikon D90 Canfield Epiflash erfasst.

Nach zwei Tagen wurde die frisch gewaschene vom ersten Tag definierte Fläche erneut untersucht und entsprechend erfasst.

Bestimmung der Haarformel über die Haardichte, Dichte des Haars in der anagenen Phase und Dichte des Haars in der telogenen Phase, anschließend zweimal täglich Anwenden der Zubereitung 1.

Nach 20 Wochen wurden die Probanden erneut untersucht, die am ersten Tag definierte Fläche wurde lokalisiert, das Haar wurde rasiert und eine Bildanalyse der definierten Fläche durchgeführt.

Am zweiten Tag der Woche 20 wurden die Probanden erneut untersucht, auf das frisch gewaschene Haar waren keine weiteren Produkte aufgetragen worden. Die am ersten Tag definierte Fläche wurde lokalisiert, die Haardichte, Dichte des Haars in der anagenen Phase und Dichte des Haars in der telogenen Phase wurde bestimmt.

Die Messungen wurden mittels Fototrichogramm durchgeführt, nämlich mit einer Nikon D80-Kamera verbunden mit Canfield Epiflash ausgestattet mit zoom lenses. Es wurden folgende Parameter evaluiert:
- Kapillardichte: Gesamtzahl des Haars an der untersuchten Fläche pro cm²
- Dichte des Haars in der anagenen Phase (A) Anteil in % und Dichte des Haars in der telogenen Phase (T)
- Wachstumsfaktor (A/T)

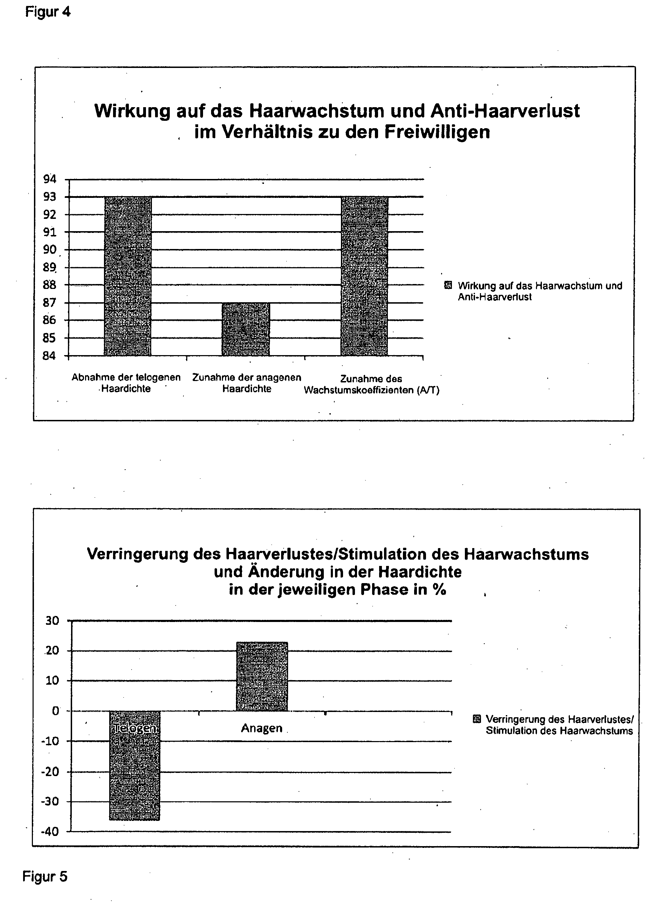

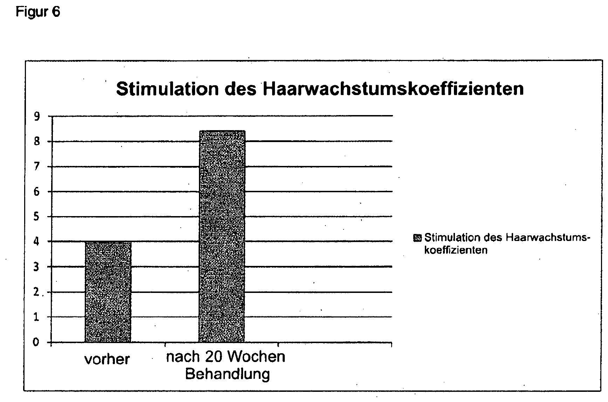

Es wurde festgestellt, dass nach Anwendung der Zubereitung 1 über 20 Wochen zweimal am Tag eine deutliche Steigerung des Wachstumsfaktors von 93 % beobachtet wurde. 93 % der Probanden zeigte eine positive Wirkung der Zubereitung 1. Die Ergebnisse sind in den Figuren 4, 5 und 6 dargestellt.

## Patentansprüche

1. Haarwuchsmittel auf Basis einer wässrigen, alkoholischen und/oder wässrig-alkoholischen Lösung enthaltend
A) 0,001 bis 0,99 Gew.-Anteile, bezogen auf 100 Gewichtsteile des fertigen Mittels, Menthol,
B) 0,1 bis 3 Gew.-Anteile, bezogen auf 100 Gewichtsteile des fertigen Mittels, Presssaft von Bambus oder Bambus-Extrakt,
C) 5 bis 10 Gew.-Anteile, bezogen auf 100 Gewichtsteile des fertigen Mittels, Hülsenfrüchte-Extrakt oder Presssaft von Hülsenfrüchten,
D) 2 bis 10 Gew.-Anteile, bezogen auf 100 Gewichtsteile des fertigen Mittels Kräuterextrakte oder Presssaft aus Kräutern.

2. Mittel nach Anspruch 1, **dadurch gekennzeichnet, dass** die Hülsenfrüchte der Komponente C ausgewählt sind aus Bohnen, Erbsen, Erdnüsse, Ginster, Kichererbsen, Klee, Linsen, Lupinen, Sojabohnen, und/oder Wicke.

3. Mittel nach einem der Ansprüche 1 oder 2, **dadurch gekennzeichnet, dass** die Kräuter der Komponente D ausgewählt sind aus Angelikawurzel, Andornkraut, Basilikum, Lavendel, Oregano, Quendel, Rosmarin und/oder Thymian.

4. Mittel nach einem der Ansprüche 1 bis 3, **dadurch gekennzeichnet, dass** der pH-Wert im sauren Bereich, insbesondere zwischen 4,5 und 6,5 liegt.

5. Mittel nach einem der Ansprüche 1 bis 4, **dadurch gekennzeichnet, dass** die Komponenten B, C und D in einer solchen Menge eingesetzt werden, dass die aktiven Komponenten insgesamt in einer Menge von 3 bis 20 Gew.-%, bezogen auf das fertige Mittel, enthalten sind.

6. Verfahren zur Herstellung eines Haarwuchsmittels nach einem der Ansprüche 1 bis 5, welches die Verfahrensschritte umfasst
a) Herstellen einer Lösung aus Menthol in Wasser, Alkohol oder einem Gemisch aus Wasser und Alkohol,
b) Einstellen des pH-Werts auf einen Bereich zwischen 4,5 und 6,5,
c) Hinzufügen der Inhaltsstoffe der Komponenten C und D und ggf. weiterer Komponenten unter Rühren.

## Claims

1. Hair growth composition based on an aqueous, alcoholic and/or aqueous alcoholic solution comprising
A) 0.001 to 0.99 parts by weight menthol, based on 100 parts by weight of the finished composition,
B) 0.1 to 3 parts by weight pressed juice of bamboo or bamboo extract, based on 100 parts by weight of the finished composition,
C) 5 to 10 parts by weight legumes extract or pressed juice of legumes, based on 100 parts by weight of the finished composition,
D) 2 to 10 parts by weight herb extracts or pressed juice of herbs, based on 100 parts by weight of the finished composition.

2. Composition according to Claim 1, **characterized in that** the legumes of component C are selected from beans, peas, peanuts, broom, chickpeas, clover, lentils, lupins, soybeans and/or vetch.

3. Composition according to either of Claims 1 or 2, **characterized in that** the herbs of component D are selected from angelica root, andorn herb, basil, lavender, oregano, wild thyme, rosemary and/or thyme.

4. Composition according to any of Claims 1 to 3, **characterized in that** the pH is in the acidic range, in particular between 4.5 and 6.5.

5. Composition according to any of Claims 1 to 4, **characterized in that** components B, C and D are used in such an amount that the active components are present in total in an amount of 3 to 20% by weight, based on the finished composition.

6. Process for producing a hair growth composition according to any of Claims 1 to 5, comprising the process steps of
a) preparing a solution of menthol in water, alcohol or a mixture of water and alcohol,
b) adjusting the pH to a range between 4.5 and 6.5,
c) adding the ingredients of components C and D and optionally further components with stirring.

## Revendications

1. Produit de croissance capillaire à base d'une solution aqueuse, alcoolique et/ou aqueuse-alcoolique contenant
A) 0,001 à 0,99 partie en poids, par rapport à 100 parties en poids du produit fini, de menthol,
B) 0,1 à 3 parties en poids, par rapport à 100 parties en poids du produit fini, de jus de pressage de bambou ou d'extrait de bambou,
C) 5 à 10 parties en poids, par rapport à 100 parties en poids du produit fini, d'extrait de légumineuses ou de jus de pressage de légumineuses,
D) 2 à 10 parties en poids, par rapport à 100 parties en poids du produit fini, d'extraits d'herbes ou de jus de pressage d'herbes.

2. Produit selon la revendication 1, **caractérisé en ce que** les légumineuses du composant C sont choisies parmi les haricots, les pois, les arachides, le genêt, les pois-chiches, le trèfle, le lin, les lupins, le soja, et/ou la vesce.

3. Produit selon l'une quelconque des revendications 1 et 2, **caractérisé en ce que** les herbes du composant D sont choisies parmi la racine d'angélique, le marrube blanc, le basilic, la lavande, l'origan, le serpolet, le romarin et/ou le thym.

4. Produit selon l'une quelconque des revendications 1 à 3, **caractérisé en ce que** la valeur de pH se situe dans le domaine acide, en particulier entre 4,5 et 6, 5.

5. Produit selon l'une quelconque des revendications 1 à 4, **caractérisé en ce que** les composants B, C et D sont utilisés en une telle quantité que les composants actifs sont contenus au total en une quantité de 3 à 20 % en poids, par rapport au produit fini.

6. Procédé pour la préparation d'un produit de croissance capillaire selon l'une quelconque des revendications 1 à 5, qui comprend les étapes de procédé suivantes
a) préparation d'une solution de menthol dans l'eau, dans de l'alcool ou un mélange d'eau et d'alcool,
b) ajustement de la valeur du pH dans un domaine compris entre 4,5 et 6,5,
c) ajout des ingrédients des composants C et D et éventuellement d'autres composants, sous agitation.
